# EUROPEAN PATENT APPLICATION

(11) **EP 4 122 495 A1**
(43) Date of publication of application: **25.01.2023**
(21) Application number: 21772065.5
(22) Date of filing: 18.03.2021
(51) Int. Cl.: A61K 45/00, A61P 9/04, C07D 401/14, C07D 403/12, C07D 403/14, A61K 31/4155, A61K 31/4439, A61K 31/506

(54) **TREATMENT OR PREVENTION METHOD FOR CHRONIC HEART FAILURE**

(30) Priority: 19.03.2020 JP 2020048798
(71) Applicant: Japan Tobacco Inc., Tokyo 105-6927 (JP)
(72) Inventor: OKUMA, Chihiro, Takatsuki-shi, Osaka 569-1125 (JP); SANO, Ryuhei, Takatsuki-shi, Osaka 569-1125 (JP); TOMIMOTO, Daisuke, Takatsuki-shi, Osaka 569-1125 (JP); NAKANE, Yoshitomi, Takatsuki-shi, Osaka 569-1125 (JP)
(74) Representative: Vossius & Partner Patentanwälte Rechtsanwälte mbB
(86) International application number: PCT/JP2021/011003
(87) International publication number: WO 2021/187548

(57) **Abstract**

One aim of the present invention is to treat or prevent chronic heart failure. Provided are: a pharmaceutical composition for treatment or prevention of chronic heart failure, comprising a compound inhibiting SGLT1 or a pharmaceutically acceptable salt thereof; and a method of treating or preventing chronic heart failure, comprising administering a compound inhibiting SGLT1 or a pharmaceutically acceptable salt thereof.

## Description

### TECHNICAL FIELD

The present invention relates to a pharmaceutical composition for treatment or prevention of chronic heart failure, comprising a compound inhibiting SGLT (Na⁺-glucose cotransporter) 1 or a pharmaceutically acceptable salt thereof, and a method of treating or preventing chronic heart failure, comprising administering a compound inhibiting SGLT1 or a pharmaceutically acceptable salt thereof.

### BACKGROUND ART

Heart failure is defined as a clinical syndrome of some form of cardiac dysfunction, i.e., failure of compensatory mechanisms of pump function due to structural and/or functional abnormalities in the heart, resulting in the appearance of dyspnea, malaise and/or edema, with a concomitant decrease in exercise tolerance, and characterized by an increase in left ventricular end-diastolic pressure. Chronic continuation of these conditions is chronic heart failure.

In chronic heart failure, fatigue during movement, cough, swelling and weight gain associated with swelling are observed. Chronic heart failure is classified, on the basis of the left ventricular ejection fraction, into heart failure with a reduced left ventricular ejection fraction (hereinafter referred to as "HFrEF") and heart failure with a preserved left ventricular ejection fraction (hereinafter referred to as "HFpEF").

HFrEF is thought to be mainly caused by left ventricular systolic dysfunction and is characterized by left ventricular enlargement in many cases. Here, enlargement refers to an increase in the inner diameter or lumen of the heart. In HFrEF, left ventricular fractional shortening is reduced.

Although standard treatments for HFrEF have been established, the common safety issues of these drugs, such as hemodynamic-related side effects such as hypotension and bradycardia, and hyperkalemia and decreased renal function have not been resolved. Moreover, HFrEF is still a disease with a poor prognosis.

Meanwhile, HFpEF is thought to be mainly caused by left ventricular diastolic dysfunction and is characterized by left ventricular hypertrophy in many cases. Here, hypertrophy refers to a condition in which the wall thickness of the heart increases.

Although diuretics and neurohumoral factor inhibitors have been used to treat HFpEF to relieve congestion and background diseases, respectively, no drug has improved the vital prognosis.

### CITATION LIST

### PATENT LITERATURE

Patent Literature 1 WO 2013/031922
Patent Literature 2 WO 2019/168096
Patent Literature 3 WO 2019/194207

### BRIEF DESCRIPTION OF DRAWINGS

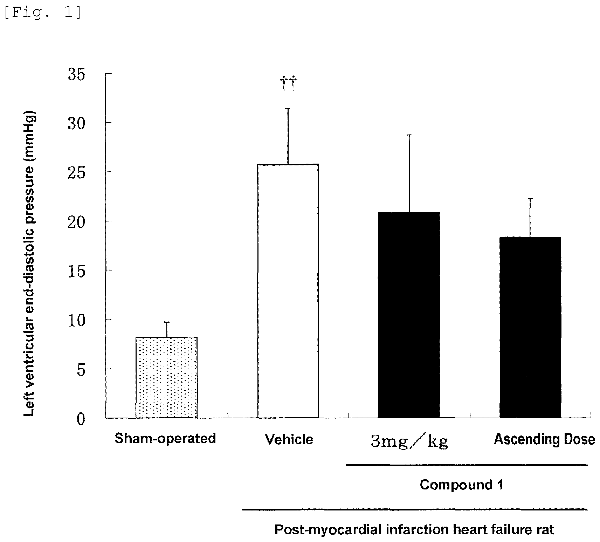

[Fig. 1] Fig. 1 shows that: the left ventricular end-diastolic pressure of the vehicle group of post-myocardial infarction heart failure rats was significantly increased in comparison with the sham-operated group; and in the post-myocardial infarction heart failure rats, the compound of Example 1 (also referred to as "Compound 1" hereinafter) showed a lower value of the left ventricular end-diastolic pressure in comparison with the vehicle.
   The symbol † † in the figure means p < 0.01 to the sham-operated group.

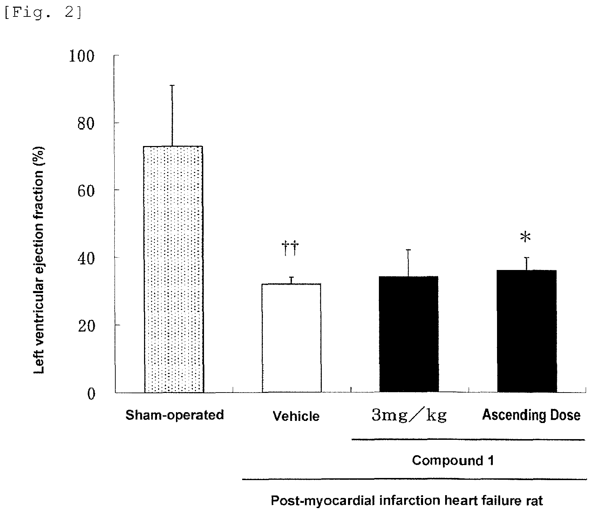

[Fig. 2] Fig. 2 shows that: the left ventricular ejection fraction of the vehicle group of post-post-myocardial infarction heart failure rats was significantly reduced in comparison with the sham-operated group; and in the post-myocardial infarction heart failure rats, Compound 1 (Ascending Dose) significantly increased the left ventricular ejection fraction in comparison with the vehicle, and Compound 1 (3mg/kg) showed a higher value of the left ventricular ejection fraction in comparison with the vehicle.
   The symbol † † in the figure means p < 0.01 to the sham-operated group. The symbol * in the figure means p < 0.05 to the vehicle group of post-myocardial infarction heart failure rats.

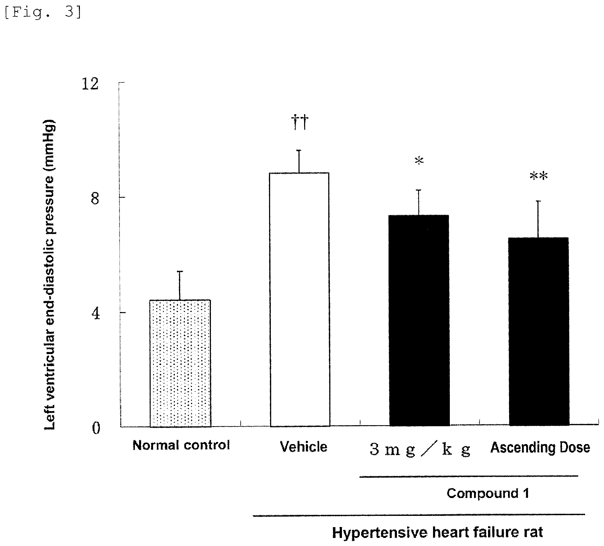

[Fig. 3] Fig. 3 shows that: the left ventricular end-diastolic pressure of the vehicle group of hypertensive heart failure rats was significantly increased in comparison with the normal control group; and in the hypertensive heart failure rats, Compound 1 significantly reduced the left ventricular end-diastolic pressure in comparison with the vehicle.
   The symbol † † in the figure means p < 0.01 to the normal control group. The symbols * and ** in the figure mean p < 0.05 and p < 0.01 to the vehicle group of hypertensive heart failure rats respectively.

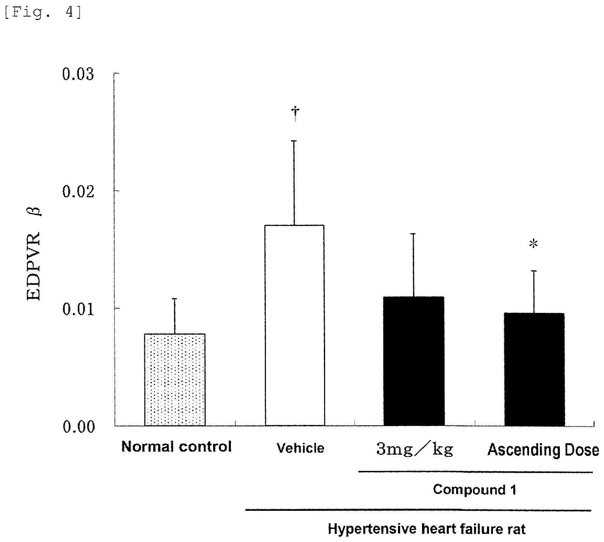

[Fig. 4] Fig. 4 shows that: the end-diastolic pressure-volume relationship (also referred to as "EDPVR" hereinafter) β of the vehicle group of hypertensive heart failure rats was significantly increased in comparison with the normal control group; and in the hypertensive heart failure rats, Compound 1 (Ascending Dose) significantly reduced EDPVRβ in comparison with the vehicle, and Compound 1 (3mg/kg) showed a lower value of the EDPVRβ in comparison with the vehicle.
   The symbol † in the figure means p < 0.05 to the normal control group. The symbol * in the figure means p < 0.05 to the vehicle group of hypertensive heart failure rats.

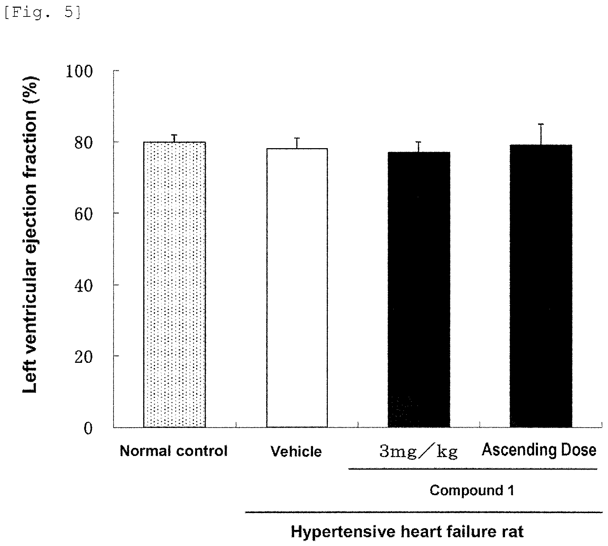

[Fig. 5] Figure 5 shows that there was no change in the left ventricular ejection fraction in all groups.

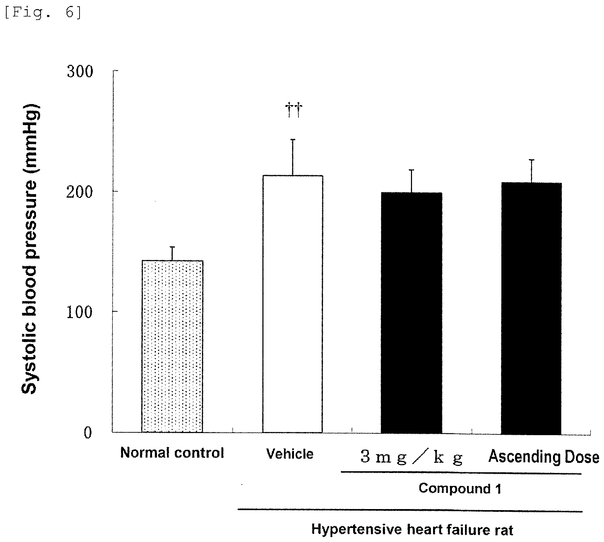

[Fig. 6] Fig. 6 shows that the systolic blood pressure of the vehicle group of hypertensive heart failure rats was significantly increased in comparison with the normal control group.
   The symbol † † in the figure means p < 0.01 to the normal control group.

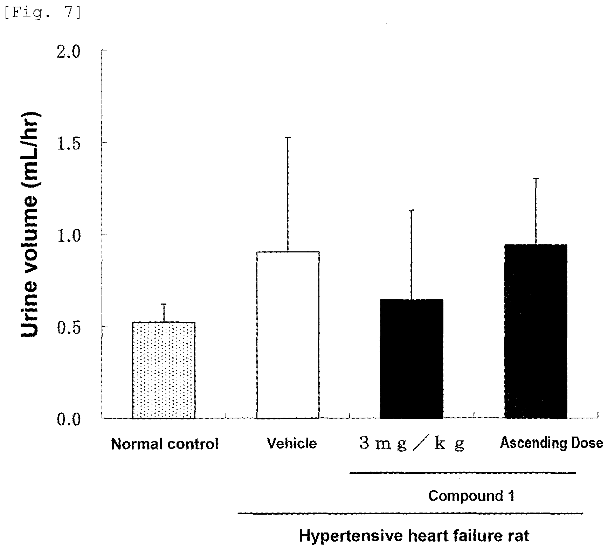

[Fig. 7] Fig. 7 shows that in the hypertensive heart failure rats, Compound 1 did not affect the urine volume in comparison with the vehicle.

### DESCRIPTION OF EMBODIMENTS

Certain embodiments are illustrated as follows.

### [Item 1]

A pharmaceutical composition for treatment or prevention of chronic heart failure, comprising a compound inhibiting SGLT1 or a pharmaceutically acceptable salt thereof.

### [Item 2]

A pharmaceutical composition for treatment or prevention of chronic heart failure, comprising a compound of Formula [I]: 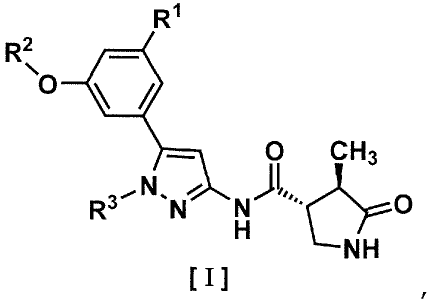 or a pharmaceutically acceptable salt thereof,
wherein R¹ is hydrogen or halogen;
R² is C₁₋₆ alkyl or halo-C₁₋₆ alkyl;
R³ is
   (1) C₁₋₆ alkyl,
   (2) halo-C₁₋₆ alkyl,
   (3) pyridyl substituted with R^{3A}, or
   (4) pyrazinyl, pyrimidinyl, or pyridazinyl, which may be optionally substituted with R^{3B};
R^{3A} is cyano, halogen, or halo-C₁₋₃ alkyl;
R^{3B} is halogen, hydroxy, C₁₋₃ alkyl, halo-C₁₋₃ alkyl, C₁₋₃ alkoxy, or -N(R⁴) (R⁵); and
R⁴ and R⁵ are each independently hydrogen or C₁₋₃ alkyl.

### [Item 3]

The pharmaceutical composition according to Item 1 or 2, wherein the compound inhibiting SGLT1 or the compound of Formula [I] or a pharmaceutically acceptable salt thereof is any one of compounds of Formulae [II] to [V]: 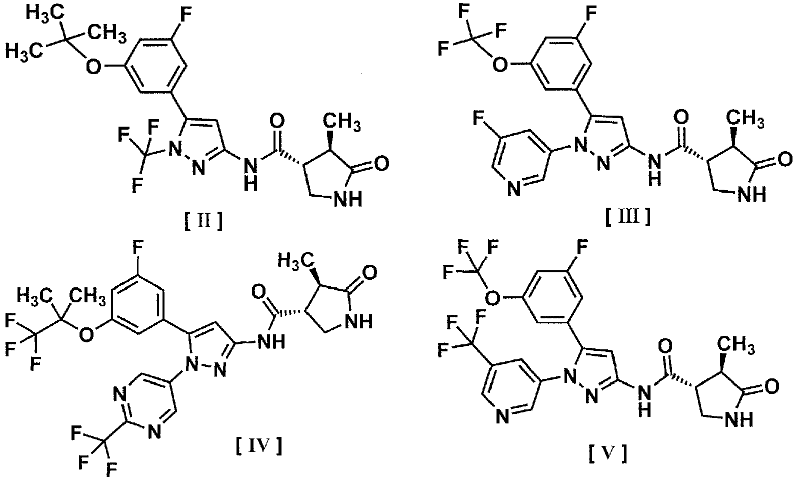 or a pharmaceutically acceptable salt thereof.

### [Item 4]

The pharmaceutical composition according to any one of Items 1 to 3, wherein the compound inhibiting SGLT1 or the compound of Formula [I] or a pharmaceutically acceptable salt thereof is a compound of Formula [II]: 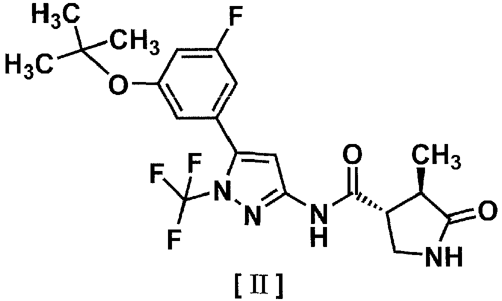 or a pharmaceutically acceptable salt thereof.

### [Item 5]

The pharmaceutical composition according to any one of Items 1 to 4, wherein the chronic heart failure is HFrEF.

### [Item 6]

The pharmaceutical composition according to any one of Items 1 to 4, wherein the chronic heart failure is HFpEF.

### [Item 7]

A method of treating or preventing chronic heart failure, comprising administering a therapeutically effective amount of a compound inhibiting SGLT1 or a compound of Formula [I] according to Item 2 or a pharmaceutically acceptable salt thereof, to a subject.

### [Item 8]

A compound inhibiting SGLT1 or a compound of Formula [I] according to Item 2 or a pharmaceutically acceptable salt thereof, for use in the treatment or prevention of chronic heart failure.

### [Item 9]

Use of a compound inhibiting SGLT1 or a compound of Formula [I] according to Item 2 or a pharmaceutically acceptable salt thereof, in the manufacture of a medicament for treatment or prevention of chronic heart failure.

A double wave line of the following: 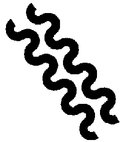 in a partial structure herein is a binding site of the structure.

The term "halogen" used herein includes, for example, fluorine, chlorine, bromine, and iodine.

The term "C₁₋₃ alkyl" used herein means a straight- or branched-chain saturated hydrocarbon group having 1 to 3 carbon atoms. The term "C₁₋₃ alkyl" includes methyl, ethyl, n-propyl, and isopropyl.

The term "C₁₋₆ alkyl" used herein means a straight- or branched-chain saturated hydrocarbon group having 1 to 6 carbon atoms. The term "C₁₋₆ alkyl" includes, for example, methyl, ethyl, n-propyl, isopropyl, n-butyl, isobutyl, sec-butyl, tert-butyl, n-pentyl, isopentyl, and n-hexyl.

The term "halo-C₁₋₃ alkyl" used herein means the above mentioned "C₁₋₃ alkyl" that is substituted with 1 to 5 halogen atoms independently selected from the group of the above mentioned "halogen". The term "halo-C₁₋₃ alkyl" includes, for example, monofluoromethyl, difluoromethyl, trifluoromethyl, 2-fluoroethyl, 2-chloroethyl, 2-bromoethyl, 1,1-difluoroethyl, 2,2,2-trifluoroethyl, pentafluoroethyl, 3-fluoropropyl, 3-chloropropyl, 1,1-difluoropropyl, and 3,3,3-trifluoropropyl.

The term "fluoro-C₁₋₃ alkyl" used herein means the above mentioned "C₁₋₃ alkyl" that is substituted with 1 to 5 fluorine atoms. The term "fluoro-C₁₋₃ alkyl" includes, for example, monofluoromethyl, difluoromethyl, trifluoromethyl, 2-fluoroethyl, 1,1-difluoroethyl, 2,2,2-trifluoroethyl, pentafluoroethyl, 3-fluoropropyl, 1,1-difluoropropyl, and 3,3,3-trifluoropropyl.

The term "halo-C₁₋₆ alkyl" used herein means the above mentioned "C₁₋₆ alkyl" that is substituted with 1 to 5 halogen atoms independently selected from the group of the above mentioned "halogen". The term "halo-C₁₋₆ alkyl" includes, for example, monofluoromethyl, difluoromethyl, trifluoromethyl, 2-fluoroethyl, 2-chloroethyl, 2-bromoethyl, 1,1-difluoroethyl, 2,2,2-trifluoroethyl, pentafluoroethyl, 3-fluoropropyl, 3-chloropropyl, 1,1-difluoropropyl, 3,3,3-trifluoropropyl, 4,4,4-trifluorobutyl, 5,5,5-trifluoropentyl, and 6,6,6-trifluorohexyl.

The term "fluoro-C₁₋₆ alkyl" used herein means the above mentioned "C₁₋₆ alkyl" that is substituted with 1 to 5 fluorine atoms. The term "fluoro-C₁₋₆ alkyl" includes, for example, monofluoromethyl, difluoromethyl, trifluoromethyl, 2-fluoroethyl, 1,1-difluoroethyl, 2,2,2-trifluoroethyl, pentafluoroethyl, 3-fluoropropyl, 1,1-difluoropropyl, 3,3,3-trifluoropropyl, 4,4,4-trifluorobutyl, 5,5,5-trifluoropentyl, and 6,6,6-trifluorohexyl.

The term "C₁₋₃ alkoxy" used herein means a group wherein the above mentioned "C₁₋₃ alkyl" binds to an oxygen atom. The term "C₁₋₃ alkoxy" includes methoxy, ethoxy, n-propoxy, and isopropoxy.

The term "pyridyl" used herein means any one of the following formulae.

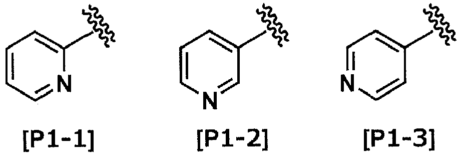

The term "pyrazinyl" used herein means the following formula.

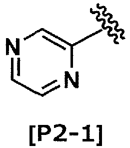

The term "pyrimidinyl" used herein means any one of the following formulae.

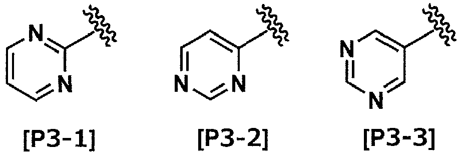

The term "pyridazinyl" used herein means any one of the following formulae.

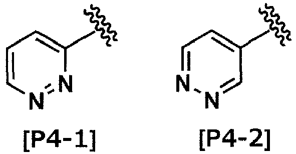

The term "substitute" used herein includes any chemically acceptable substitution. For example, the term "pyridyl substituted with R^{3A}" used herein means any one of the following formulae.

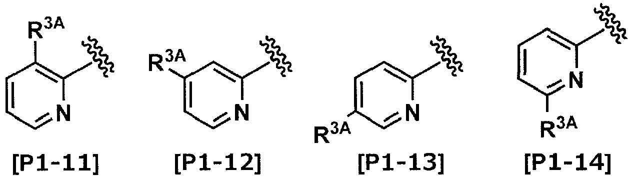

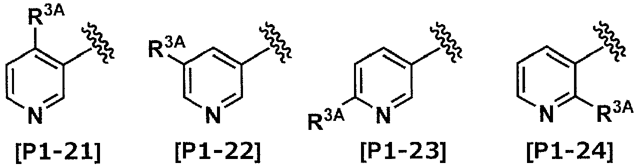

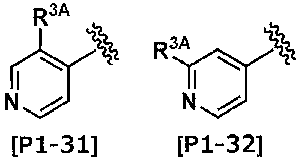

Each substituent of a compound of Formula [I] includes embodiments illustrated as below for each substituent, and a compound of Formula [I] includes any combinations of these embodiments for each substituent.

In one embodiment, R¹ is halogen. In another embodiment, R¹ is fluorine.

In one embodiment, R² is C₁₋₆ alkyl or fluoro-C₁₋₆ alkyl. In another embodiment, R² is C₁₋₆ alkyl. In still another embodiment, R² is fluoro-C₁₋₃ alkyl.

In one embodiment, R³ is
(1) halo-C₁₋₆ alkyl,
(2) pyridyl substituted with R^{3A}, or
(3) pyrazinyl or pyrimidinyl, which may be optionally substituted with R^{3B}.

In another embodiment, R³ is selected from the group consisting of halo-C₁₋₆ alkyl and groups of Formulae [H1] to [H14] .

In still another embodiment, R³ is halo-C₁₋₆ alkyl, or a group of Formula [H2] or [H8].

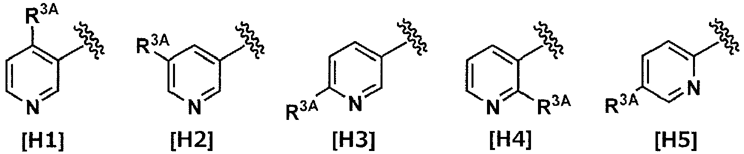

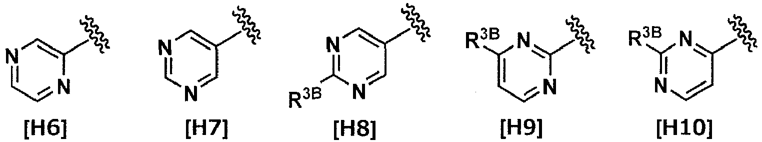

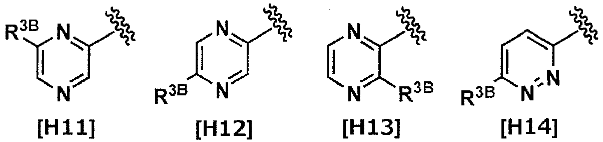

In one embodiment, R^{3A} is halogen or halo-C₁₋₃ alkyl. In another embodiment, R^{3A} is fluorine or fluoro-C₁₋₃ alkyl.

In one embodiment, R^{3B} is halogen or halo-C₁₋₃ alkyl. In another embodiment, R^{3B} is fluoro-C₁₋₃ alkyl.

In one embodiment, R⁴ and R⁵ are each independently C₁₋₃ alkyl.

In one embodiment, a compound of Formula [I] is a compound of Formula [II] or [III]: 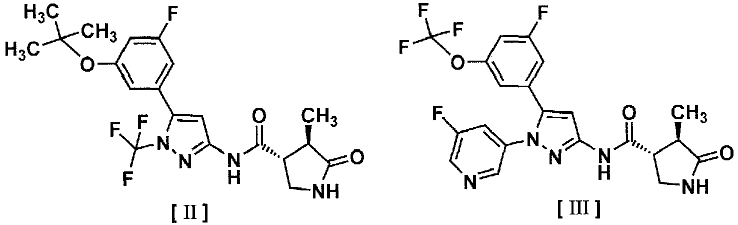 In another embodiment, a compound of Formula [I] is a compound of Formula [II]. In still another embodiment, a compound of Formula [I] is a compound of Formula [III] monohydrate, i.e., a compound of Formula [VI]:

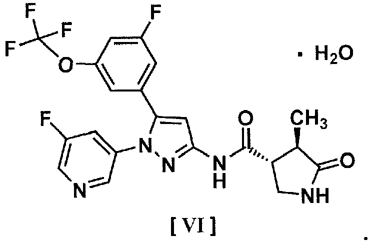

The term "pharmaceutically acceptable salt" includes any salts known in the art that are not associated with excessive toxicity. Such a pharmaceutically acceptable salt includes, specifically, salts with inorganic acids, salts with organic acids, salts with inorganic bases, and salts with organic bases. Various forms of pharmaceutically acceptable salts are well known in the art and are described in, for example, the following references:
(a) Berge et al., J. Pharm. Sci., 66, p1-19 (1977);
(b) Stahl et al., "Handbook of Pharmaceutical Salt: Properties, Selection, and Use" (Wiley-VCH, Weinheim, Germany, 2002);
(c) Paulekuhn et al., J. Med. Chem., 50, p6665-6672 (2007).

A compound of Formula [I] may be reacted with an inorganic acid, organic acid, inorganic base, or organic base according to methods known *per se* to give each corresponding pharmaceutically acceptable salt thereof.

Such a salt with inorganic acid includes a salt with hydrofluoric acid, hydrochloric acid, hydrobromic acid, hydroiodic acid, nitric acid, phosphoric acid, and sulfuric acid. Such a salt preferably includes a salt with hydrochloric acid, nitric acid, sulfuric acid, phosphoric acid, and hydrobromic acid.

Such a salt with organic acid includes a salt with acetic acid, adipic acid, alginic acid, 4-aminosalicylic acid, anhydromethylenecitric acid, benzoic acid, benzenesulfonic acid, calcium edetate, camphor acid, camphor-10-sulfonic acid, carbonic acid, citric acid, edetic acid, ethane-1,2-disulfonic acid, dodecylsulfuric acid, ethanesulfonic acid, fumaric acid, glucoheptonic acid, gluconic acid, glucuronic acid, glucoheptonic acid, glycollylarsanilic acid, hexylresorcinol acid, hydroxynaphthoic acid, 2-hydroxy-1-ethanesulfonic acid, lactic acid, lactobionic acid, malic acid, maleic acid, mandelic acid, methanesulfonic acid, methylsulfuric acid, methylnitric acid, methylenebis(salicylic acid), galactaric acid, naphthalene-2-sulfonic acid, 2-naphthoic acid, 1,5-naphthalenedisulfonic acid, oleic acid, oxalic acid, pamoic acid, pantothenic acid, pectic acid, picric acid, propionic acid, polygalacturonic acid, salicylic acid, stearic acid, succinic acid, tannic acid, tartaric acid, teoclic acid, thiocyanic acid, trifluoroacetic acid, p-toluenesulfonic acid, undecanoic acid, aspartic acid, and glutamic acid. Such a salt preferably includes a salt with oxalic acid, maleic acid, citric acid, fumaric acid, lactic acid, malic acid, succinic acid, tartaric acid, acetic acid, trifluoroacetic acid, benzoic acid, glucuronic acid, oleic acid, pamoic acid, methanesulfonic acid, benzenesulfonic acid, p-toluenesulfonic acid, and 2-hydroxy-1-ethanesulfonic acid.

Such a salt with inorganic base includes a salt with lithium, sodium, potassium, magnesium, calcium, barium, aluminum, zinc, bismuth, and ammoinum. Such a salt preferably includes a salt with sodium, potassium, calcium, magnesium, and zinc.

Such a salt with organic base includes a salt with arecoline, betaine, choline, clemizole, ethylenediamine, N-methylglucamine, N-benzylphenethylamine, tris(hydroxymethyl)methylamine, arginine, and lysine. Such a salt preferably includes a salt with tris(hydroxymethyl)methylamine, N-methylglucamine, and lysine.

A compound inhibiting SGLT1 or a pharmaceutically acceptable salt thereof (hereinafter also referred to as an SGLT1 inhibitor) may be any substance that inhibits SGLT1, and includes small molecules, nucleic acids, polypeptides, proteins, antibodies, vaccines, etc. SGLT1 inhibitory activity may be measured by methods known to those of ordinary skill in the art and may be calculated based on the intracellular uptake of the labeled form of α-methyl-D-glucopyranoside (¹⁴C-AMG) transported by SGLT1, for example, according to the evaluation method described in any of Patent Literatures 1 to 3.

In one embodiment, the SGLT1 inhibitor is a compound of Formula [I]: 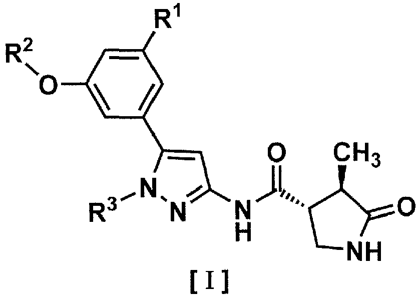 or a pharmaceutically acceptable salt thereof,
wherein each symbol has the same meaning as defined above.

Compounds encompassed by Formula (I) can be prepared by methods known to those skilled in the art and may be prepared, for example, by any of the methods described in Patent Literatures 1-3. Moreover, the SGLT1 inhibitory activities of the compounds encompassed by Formula [I] have been confirmed by these literatures.

As used herein, an SGLT1 inhibitor may be used in combination with other drugs, such as a compound inhibiting SGLT2 or a pharmaceutically acceptable salt thereof (hereinafter also referred to as an SGLT2 inhibitor) to treat and/or prevent chronic heart failure. An SGLT2 inhibitor herein may be any substance that inhibits SGLT2, and includes small molecules, nucleic acids, polypeptides, proteins, antibodies, vaccines, etc. In one embodiment, an SGLT2 inhibitor is a substance with a function to inhibit reuptake of glucose from the urine to increase the excretion amount of sugar in the urine so that the blood glucose level may be reduced.

An SGLT1 inhibitor, e.g., a compound of Formula [I] or a pharmaceutically acceptable salt thereof and an SGLT2 inhibitor may exist in their solvate forms. The term "solvate" means a compound where a solvent molecule is coordinated with, for example, a compound of Formula [I] or a pharmaceutically acceptable salt thereof. The solvate may be a pharmaceutically acceptable solvate; and includes, for example, a hydrate, an ethanolate, and a dimethylsulfoxide solvate of a compound of Formula [I] or a pharmaceutically acceptable salt thereof. The solvate specifically includes a hemihydrate, monohydrate, dihydrate, and monoethanolate of a compound of Formula [I]; and a monohydrate of sodium salt of a compound of Formula [I] and a 2/3 ethanolate of dihydrochloride salt thereof. These solvates may be obtained according to any of the known methods. For example, a compound of Formula [III] may exist as its monohydrate as seen in the following Formula [VI] .

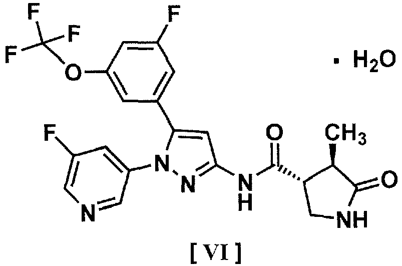

A compound of Formula [I] may be labelled with an isotope such as ²H, ³H, ¹⁴C, and ³⁵S.

A compound of Formula [I] or a pharmaceutically acceptable salt thereof is preferably a compound of Formula [I] or a pharmaceutically acceptable salt thereof that is substantively purified, and more preferably a compound of Formula [I] or a pharmaceutically acceptable salt thereof that is purified into a purity of 80% or more.

Inhibiting SGLT1 means that the function of SGLT1 is inhibited so as to disappear or reduce its activity. Preferably, inhibiting SGLT1 means inhibiting human SGLT1. The inhibition of the function of SGLT1, or the disappearance or reduction of its activity is preferably carried out in human clinical indications. In one embodiment, inhibiting SGLT1 may suppress the increase in left ventricular end-diastolic pressure and/or may increase left ventricular ejection fraction, thereby improving HFrEF and resulting in a preventive or therapeutic effect on chronic heart failure. In another aspect, inhibiting SGLT1 may lower left ventricular end-diastolic pressure and/or may lower EDPVRβ, thereby improving HFpEF and resulting in a preventive or therapeutic effect on chronic heart failure. An SGLT1 inhibitor may improve HFrEF or HFpEF, or both.

Inhibiting SGLT2 means that the function of SGLT2 is inhibited so as to disappear or reduce its activity. Preferably, inhibiting SGLT2 means inhibiting human SGLT2. The inhibition of the function of SGLT2, or the disappearance or reduction of its activity is preferably carried out in human clinical indications.

The SGLT2 inhibitor herein includes, for example, glycosides and salts thereof and solvates thereof. The glycosides herein are those compounds wherein sugars or sugar derivatives glycosidically bind to aglycone moieties (e.g., through a C-glycosidic bond or O-glycosidic bond) and the sugars or sugar derivatives are those having the following structure: 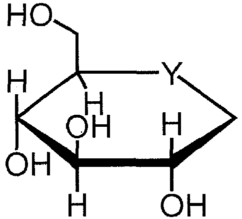 wherein Y is O or S and a glycosidic bond is formed on the carbon atom at the 1-position.

The SGLT2 inhibitor herein includes, for example, the following compounds. For the convenience, trivial names are used herein.

**[Table 1]**

| Trivial name | Generic name |
|---|---|
| Dapagliflozin | Dapagliflozin propylene glycol hydrate |
| Ipragliflozin | Ipragliflozin L-proline |
| Tofogliflozin | Tofogliflozin hydrate |
| Empagliflozin | Empagliflozin |
| Canagliflozin | Canagliflozin hydrate |
| Luseogliflozin | Luseogliflozin hydrate |

**[Table 2]**

| Trivial name | Structure |
|---|---|
| Dapagliflozin | 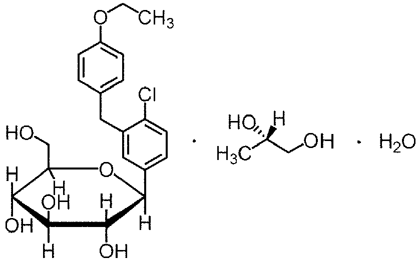 |
| Ipragliflozin | 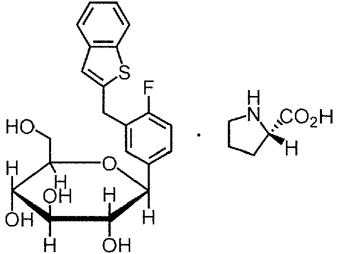 |
| Tofogliflozin | 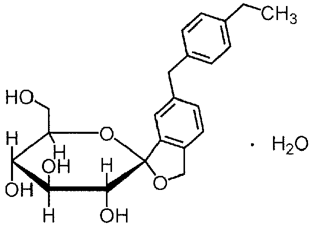 |
| Empagliflozin | 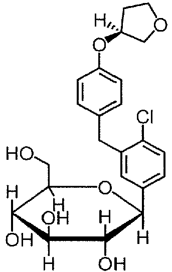 |
| Canagliflozin | 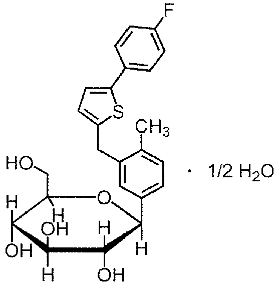 |
| Luseogliflozin | 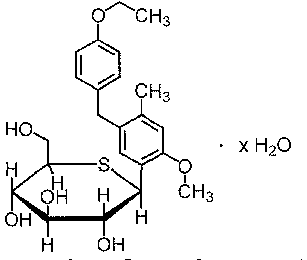 |
| | In the formula, x is an arbitrary number. |

In one embodiment, an SGLT1 inhibitor may be used in treatment and/or prevention of chronic heart failure by administration thereof to a subject in combination with an SGLT2 inhibitor.

In another embodiment, provided is a medicament for use in treatment or prevention of chronic heart failure, comprising an SGLT1 inhibitor, which comprises administration of the SGLT1 inhibitor in combination with an SGLT2 inhibitor.

In still another embodiment, provided is a medicament for use in treatment or prevention of chronic heart failure, comprising an SGLT2 inhibitor, which comprises administration of the SGLT2 inhibitor in combination with an SGLT1 inhibitor.

In still another embodiment, provided is a medicament for use in treatment or prevention of chronic heart failure, comprising an SGLT1 inhibitor, which comprises administration of the medicament to a subject undergoing treatment with an SGLT2 inhibitor.

In still another embodiment, provided is a medicament for use in treatment or prevention of chronic heart failure, comprising an SGLT2 inhibitor, which comprises administration of the medicament to a subject undergoing treatment with an SGLT1 inhibitor.

The phrase "used in combination (or combination use)" used herein means, for example, administering an SGLT1 inhibitor and an SGLT2 inhibitor in any order to a subject. Each drug has each particular mode of action, and combination use of these drugs may provide an additive or synergistic therapeutic or preventive effect. In one embodiment, combination use where multiple drugs with different modes of action are used may reduce the dosage amount of each drug compared to the case where each drug is used alone, and may reduce side effects specific to each drug. The side effects herein include, for example, hypoglycemia, body weight gain, dehydration, excessive urination, and urinary frequency. In one embodiment, an SGLT1 inhibitor and an SGLT2 inhibitor may be administered to a subject concurrently, sequentially, or with a certain interval, e.g., within 30 minutes, within one hour, within two hours, and within four hours, together or separately in any order. The one drug may be administered while a therapeutically effective amount of the active ingredient comprised in the other drug administered first exists in the body of a subject when said one drug is administered to the subject. In another embodiment, an SGLT1 inhibitor may be administered to a subject in a single combined formulation wherein the SGLT1 inhibitor is comprised in combination with an SGLT2 inhibitor. The ratios of these drugs to be administered or blended may be optionally selected depending on subjects to be administered, administration routes, subject diseases, symptoms, severity of diseases, and combinations thereof. For example, when the subjects to be administered are a human, 0.01 to 1000 parts by weight of an SGLT2 inhibitor may be used for one part by weight of an SGLT1 inhibitor.

In one embodiment, combination use of an SGLT1 inhibitor with an SGLT2 inhibitor includes use of a compound of Formula [I] or a pharmaceutically acceptable salt thereof in combination with glycoside or a salt thereof or a solvate thereof.

In another embodiment, combination use of an SGLT1 inhibitor with an SGLT2 inhibitor includes use of a compound of Formula [II] or a pharmaceutically acceptable salt thereof in combination with glycoside or a salt thereof or a solvate thereof.

In one embodiment, combination use of an SGLT1 inhibitor with an SGLT2 inhibitor includes, for example: use of a compound of Formula [I] or a pharmaceutically acceptable salt thereof in combination with dapagliflozin, use of a compound of Formula [I] or a pharmaceutically acceptable salt thereof in combination with ipragliflozin, use of a compound of Formula [I] or a pharmaceutically acceptable salt thereof in combination with tofogliflozin, use of a compound of Formula [I] or a pharmaceutically acceptable salt thereof in combination with empagliflozin, use of a compound of Formula [I] or a pharmaceutically acceptable salt thereof in combination with canagliflozin, and use of a compound of Formula [I] or a pharmaceutically acceptable salt thereof in combination with luseogliflozin.

In another embodiment, combination use of an SGLT1 inhibitor with an SGLT2 inhibitor includes: use of a compound of Formula [II] or a pharmaceutically acceptable salt thereof in combination with dapagliflozin, use of a compound of Formula [II] or a pharmaceutically acceptable salt thereof in combination with ipragliflozin, use of a compound of Formula [II] or a pharmaceutically acceptable salt thereof in combination with tofogliflozin, use of a compound of Formula [II] or a pharmaceutically acceptable salt thereof in combination with empagliflozin, use of a compound of Formula [II] or a pharmaceutically acceptable salt thereof in combination with canagliflozin, and use of a compound of Formula [II] or a pharmaceutically acceptable salt thereof in combination with luseogliflozin.

The "combination use (or used in combination)" used herein includes a combination use of a compound of Formula [I] or a pharmaceutically acceptable salt thereof and dapagliflozin.

The "combination use (or used in combination)" used herein includes a combination use of a compound of Formula [I] or a pharmaceutically acceptable salt thereof and empagliflozin.

The "combination use (or used in combination)" used herein includes a combination use of a compound of Formula [II] or a pharmaceutically acceptable salt thereof and dapagliflozin.

The "combination use (or used in combination)" used herein includes a combination use of a compound of Formula [II] or a pharmaceutically acceptable salt thereof and empagliflozin.

The drug used herein means either SGLT1 inhibitors or SGLT2 inhibitors. Administering a drug to a subject who is subject to treatment with another drug is one embodiment of combination use; for example, when a drug is administered to a subject, the combination use includes administration of the drug to the subject while a therapeutically effective amount of an active ingredient included in another drug that has been administered is present in the body of the subject.

A therapeutically effective amount of an SGLT1 inhibitor used herein may vary depending on subjects to be administered, administration routes, intended diseases, symptoms, severity of diseases, and combination thereof. When a human (60 kg of body weight) is orally administered, the lower limit of the therapeutically effective amount includes, for example, about 0.01 mg, about 0.1 mg, about 0.5 mg, about 1 mg, about 10 mg, about 20 mg, or about 50 mg per day, and the upper limit of the therapeutically effective amount includes, for example, about 1 mg, about 5 mg, about 10 mg, about 20 mg, about 50 mg, about 100 mg, about 200 mg, about 500 mg, or about 1000 mg per day.

The frequency of administration for an SGLT1 inhibitor, and pharmaceutical compositions herein includes once, twice, thrice, or more per day.

The term "treatment" used herein includes the amelioration of conditions, prevention of aggravation, maintenance of remission, and prevention of relapse. For example, the treatment of chronic heart failure includes recovery and amelioration of cardiac function, specifically includes decrease in left ventricular end-diastolic pressure, increase in left ventricular ejection fraction, and decrease in EDPVR (end-diastolic pressure-volume relationship) β.

The term "prevention" used herein includes suppressing the onset of conditions. For example, the prevention of chronic heart failure includes maintenance of cardiac function, specifically, suppression of elevated left ventricular end-diastolic pressure, maintenance of left ventricular ejection fraction, and maintenance of systolic blood pressure.

"Chronic heart failure" as used herein, often also referred to as congestive heart failure, consists of HFrEF and HFpEF. "HFrEF" is often also referred to as systolic heart failure and includes, for example, post-myocardial infarction heart failure. "HFpEF" is often also referred to as diastolic heart failure and includes, for example, hypertensive heart failure.

"HFrEF", as used herein, is defined as heart failure with a reduced left ventricular ejection fraction and, more specifically, heart failure with a left ventricular ejection fraction of less than 45%. In one embodiment, HFrEF includes heart failure with a left ventricular ejection fraction of less than 40%. In another embodiment, HFrEF includes heart failure with a left ventricular ejection fraction of less than 35%.

In one embodiment, HFrEF includes HFrEF caused by coronary artery disease. Coronary artery disease includes, for example, ischemic heart disease, specifically angina pectoris and myocardial infarction. Angina pectoris is a disease caused by narrowing of the coronary arteries due to arteriosclerosis etc. and, for example, excessive absorption of glucose into the blood may be a contributing factor. Myocardial infarction is a disease caused by clogging of the coronary arteries, and, for example, excessive absorption of glucose into cardiomyocytes may be a contributing factor.

"HFpEF" as used herein is defined as heart failure with a preserved left ventricular ejection fraction and, more specifically, heart failure with a left ventricular ejection fraction of 450 or higher. In one embodiment, HFpEF includes heart failure with a left ventricular ejection fraction of 500 or higher. In one embodiment, HFpEF includes HFpEF caused by hypertension.

Left ventricular ejection fraction is the ratio of stroke volume (blood volume pumped by the heart with each heartbeat) divided by the left ventricular end-diastolic volume when the heart dilates. Left ventricular ejection fraction can be obtained, for example, using an ultrasonograph.

EDPVR shows the relationship between end-diastolic volume and end-diastolic pressure, which can be approximated by an exponential curve. It is indicated that the higher the EDPVRβ, which is a slope of the curve, is, the more difficulty the heart has in dilatation. That is, EDPVRβ is one of the indicators of left ventricular diastolic function. For example, when EDPVRβ is over 0.015, it means that the left ventricular diastolic function is reduced.

EDPVR can be obtained using, for example, a pressure-volume measuring catheter system.

In one embodiment, the present invention provides a pharmaceutical composition for treatment or prevention of chronic heart failure, comprising an SGLT1 inhibitor.

In one embodiment, the present invention provides a pharmaceutical composition for treatment or prevention of chronic heart failure caused by coronary artery disease or hypertension, comprising an SGLT1 inhibitor. In another embodiment, the present invention provides a pharmaceutical composition for treatment or prevention of chronic heart failure caused by angina pectoris, myocardial infarction or hypertension, comprising an SGLT1 inhibitor.

In one embodiment, the present invention provides a pharmaceutical composition for reducing left ventricular end-diastolic pressure in a subject with a left ventricular end-diastolic pressure higher than a normal range, comprising an SGLT1 inhibitor. In one embodiment, the normal range of left ventricular end-diastolic pressure is 4 to 8 mmHg.

In one embodiment, the present invention provides a pharmaceutical composition for treatment or prevention of HFrEF, comprising an SGLT1 inhibitor.

In one embodiment, the present invention provides a pharmaceutical composition for treatment or prevention of HFrEF caused by coronary artery disease, comprising an SGLT1 inhibitor. In another embodiment, the present invention provides a pharmaceutical composition for treatment or prevention of HFrEF caused by angina pectoris or myocardial infarction, comprising an SGLT1 inhibitor.

In one embodiment, the present invention provides a pharmaceutical composition for increasing left ventricular ejection fraction in a subject whose left ventricular ejection fraction is reduced to less than 450, comprising an SGLT1 inhibitor.

In one embodiment, the present invention provides a pharmaceutical composition for treatment or prevention of HFpEF, comprising an SGLT1 inhibitor.

In one embodiment, the present invention provides a pharmaceutical composition for treatment or prevention of HFpEF caused by hypertension, comprising an SGLT1 inhibitor.

In one embodiment, the present invention provides a pharmaceutical composition for reducing EDPVR (end-diastolic pressure-volume relationship) β in a subject with a high EDPVR (end-diastolic pressure-volume relationship) β, comprising an SGLT1 inhibitor.

In one embodiment, the present invention provides a pharmaceutical composition for treatment or prevention of chronic heart failure, comprising a compound of Formula [I] or a pharmaceutically acceptable salt thereof.

In one embodiment, the present invention provides a pharmaceutical composition for treatment or prevention of chronic heart failure caused by coronary artery disease or hypertension, comprising a compound of Formula [I] or a pharmaceutically acceptable salt thereof. In another embodiment, the present invention provides a pharmaceutical composition for treatment or prevention of chronic heart failure caused by angina pectoris, myocardial infarction or hypertension, comprising a compound of Formula [I] or a pharmaceutically acceptable salt thereof.

In one embodiment, the present invention provides a pharmaceutical composition for reducing left ventricular end-diastolic pressure in a subject with a left ventricular end-diastolic pressure higher than a normal range, comprising a compound of Formula [I] or a pharmaceutically acceptable salt thereof.

In one embodiment, the present invention provides a pharmaceutical composition for treatment or prevention of HFrEF, comprising a compound of Formula [I] or a pharmaceutically acceptable salt thereof.

In one embodiment, the present invention provides a pharmaceutical composition for treatment or prevention of HFrEF caused by coronary artery disease, comprising a compound of Formula [I] or a pharmaceutically acceptable salt thereof. In another embodiment, the present invention provides a pharmaceutical composition for treatment or prevention of HFrEF caused by angina pectoris or myocardial infarction, comprising a compound of Formula [I] or a pharmaceutically acceptable salt thereof.

In one embodiment, the present invention provides a pharmaceutical composition for increasing left ventricular ejection fraction in a subject whose left ventricular ejection fraction is reduced to less than 350, comprising a compound of Formula [I] or a pharmaceutically acceptable salt thereof.

In one embodiment, the present invention provides a pharmaceutical composition for treatment or prevention of HFpEF, comprising a compound of Formula [I] or a pharmaceutically acceptable salt thereof.

In one embodiment, the present invention provides a pharmaceutical composition for treatment or prevention of HFpEF caused by hypertension, comprising a compound of Formula [I] or a pharmaceutically acceptable salt thereof.

In one embodiment, the present invention provides a pharmaceutical composition for reducing EDPVR (end-diastolic pressure-volume relationship) β in a subject whose EDPVR (end-diastolic pressure-volume relationship) β is higher than 0.015, comprising a compound of Formula [I] or a pharmaceutically acceptable salt thereof.

In one embodiment, the present invention provides a pharmaceutical composition for treatment or prevention of chronic heart failure, comprising a compound of Formula [II] or a pharmaceutically acceptable salt thereof.

In one embodiment, the present invention provides a pharmaceutical composition for treatment or prevention of chronic heart failure caused by coronary artery disease or hypertension, comprising a compound of Formula [II] or a pharmaceutically acceptable salt thereof. In another embodiment, the present invention provides a pharmaceutical composition for treatment or prevention of chronic heart failure caused by angina pectoris, myocardial infarction or hypertension, comprising a compound of Formula [II] or a pharmaceutically acceptable salt thereof.

In one embodiment, the present invention provides a pharmaceutical composition for reducing left ventricular end-diastolic pressure in a subject with a left ventricular end-diastolic pressure higher than a normal range, comprising a compound of Formula [II] or a pharmaceutically acceptable salt thereof.

In one embodiment, the present invention provides a pharmaceutical composition for treatment or prevention of HFrEF, comprising a compound of Formula [II] or a pharmaceutically acceptable salt thereof.

In one embodiment, the present invention provides a pharmaceutical composition for treatment or prevention of HFrEF caused by coronary artery disease, comprising a compound of Formula [II] or a pharmaceutically acceptable salt thereof. In another embodiment, the present invention provides a pharmaceutical composition for treatment or prevention of HFrEF caused by angina pectoris or myocardial infarction, comprising a compound of Formula [II] or a pharmaceutically acceptable salt thereof.

In one embodiment, the present invention provides a pharmaceutical composition for increasing left ventricular ejection fraction in a subject whose left ventricular ejection fraction is reduced to less than 350, comprising a compound of Formula [II] or a pharmaceutically acceptable salt thereof.

In one embodiment, the present invention provides a pharmaceutical composition for treatment or prevention of HFpEF, comprising a compound of Formula [II] or a pharmaceutically acceptable salt thereof.

In one embodiment, the present invention provides a pharmaceutical composition for treatment or prevention of HFpEF caused by hypertension, comprising a compound of Formula [II] or a pharmaceutically acceptable salt thereof.

In one embodiment, the present invention provides a pharmaceutical composition for reducing EDPVR (end-diastolic pressure-volume relationship) β in a subject whose EDPVR (end-diastolic pressure-volume relationship) β is higher than 0.015, comprising a compound of Formula [II] or a pharmaceutically acceptable salt thereof.

In one embodiment, the present invention provides a pharmaceutical composition for treatment or prevention of chronic heart failure, comprising an SGLT1 inhibitor and an SGLT2 inhibitor.

In one embodiment, the present invention provides a pharmaceutical composition for treatment or prevention of chronic heart failure caused by coronary artery disease or hypertension, comprising an SGLT1 inhibitor and an SGLT2 inhibitor. In another embodiment, the present invention provides a pharmaceutical composition for treatment or prevention of chronic heart failure caused by angina pectoris, myocardial infarction or hypertension, comprising an SGLT1 inhibitor and an SGLT2 inhibitor.

In one embodiment, the present invention provides a pharmaceutical composition for reducing left ventricular end-diastolic pressure in a subject with a left ventricular end-diastolic pressure higher than a normal range, comprising an SGLT1 inhibitor and an SGLT2 inhibitor.

In one embodiment, the present invention provides a pharmaceutical composition for treatment or prevention of HFrEF, comprising an SGLT1 inhibitor and an SGLT2 inhibitor.

In one embodiment, the present invention provides a pharmaceutical composition for treatment or prevention of HFrEF caused by coronary artery disease, comprising an SGLT1 inhibitor and an SGLT2 inhibitor. In another embodiment, the present invention provides a pharmaceutical composition for treatment or prevention of HFrEF caused by angina pectoris or myocardial infarction, comprising an SGLT1 inhibitor and an SGLT2 inhibitor.

In one embodiment, the present invention provides a pharmaceutical composition for increasing left ventricular ejection fraction in a subject whose left ventricular ejection fraction is reduced to less than 350, comprising an SGLT1 inhibitor and an SGLT2 inhibitor.

In one embodiment, the present invention provides a pharmaceutical composition for treatment or prevention of HFpEF, comprising an SGLT1 inhibitor and an SGLT2 inhibitor.

In one embodiment, the present invention provides a pharmaceutical composition for treatment or prevention of HFpEF caused by hypertension, comprising an SGLT1 inhibitor and an SGLT2 inhibitor.

In one embodiment, the present invention provides a pharmaceutical composition for reducing EDPVR (end-diastolic pressure-volume relationship) β in a subject whose EDPVR (end-diastolic pressure-volume relationship) β is higher than 0.015, comprising an SGLT1 inhibitor and an SGLT2 inhibitor.

A pharmaceutical composition herein may be prepared from a therapeutically effective amount of SGLT1 inhibitor and at least one or more pharmaceutically acceptable carriers, and an SGLT2 inhibitor if needed, optionally followed by mixing, according to methods known in the art of medicinal preparations. The amount of SGLT1 inhibitor in the pharmaceutical composition varies depending on a factor such as dosage forms and dosage amounts and ranges, for example, from 0.1 to 100% by weight of the total amount of the composition.

A dosage form of pharmaceutical composition herein includes oral preparations such as tablets, capsules, granules, powders, lozenges, syrups, emulsions, and suspensions; and parenteral preparations such as external preparations, suppositories, injections, eye drops, nasal preparations, and pulmonary preparations.

The term "pharmaceutically acceptable carrier" includes various organic or inorganic carrier substances which are conventionally used for a component of a formulation. Such substances include, for example, excipients, disintegrants, binders, fluidizers, and lubricants for solid preparations; solvents, solubilization agents, suspending agents, tonicity agents, buffering agents, and soothing agents for liquid preparations; and bases, emulsifying agents, wetting agents, stabilizers, stabilizing agents, dispersing agents, plasticizing agents, pH adjusters, absorption promoters, gelators, antiseptic agents, bulking agents, solubilizers, solubilization agents, and suspending agents for semisolid preparations. Additives such as preserving agents, antioxidant agents, coloring agents, and sweetening agents may be further added, if needed.

Such an "excipient" includes, for example, lactose, white soft sugar, D-mannitol, D-sorbitol, corn starch, dextrin, microcrystalline cellulose, crystalline cellulose, carmellose, carmellose calcium, sodium carboxymethylstarch, low-substitiuted hydroxypropylcellulose, and gum arabic.

Such a "disintegrant" includes, for example, carmellose, carmellose calcium, carmellose sodium, sodium carboxymethylstarch, croscarmellose sodium, crospovidone, low-substituted hydroxypropylcellulose, hydroxypropylmethyl cellulose, and crystalline cellulose.

Such a "binder" includes, for example, hydroxypropylcellulose, hydroxypropylmethyl cellulose, povidone, crystalline cellulose, white soft sugar, dextrin, starch, gelatin, carmellose sodium, and gum arabic.

Such a "fluidizer" includes, for example, light anhydrous silicic acid and magnesium stearate.

Such a "lubricant" includes, for example, magnesium stearate, calcium stearate, and talc.

Such a "solvent" includes, for example, purified water, ethanol, propylene glycol, macrogol, sesame oil, corn oil, and olive oil.

Such a "solubilization agent" includes, for example, propylene glycol, D-mannitol, benzyl benzoate, ethanol, triethanolamine, sodium carbonate, and sodium citrate.

Such a "suspending agent" includes, for example, benzalkonium chloride, carmellose, hydroxypropylcellulose, propylene glycol, povidone, methylcellulose, and glyceryl monostearate.

Such a "tonicity agent" includes, for example, glucose, D-sorbitol, sodium chloride, and D-mannitol.

Such a "buffering agent" includes, for example, disodium hydrogen phosphate, sodium acetate, sodium carbonate, and sodium citrate.

Such a "soothing agent" includes, for example, benzyl alcohol.

Such a "base" includes, for example, water, oils from animals or vegetables such as olive oil, corn oil, arachis oil, sesame oil, and castor oil, lower alcohols such as ethanol, propanol, propylene glycol, 1,3-butylene glycol, and phenol, higher fatty acids and esters thereof, waxes, higher alcohol, polyhydric alcohol, hydrocarbons such as white petrolatum, liquid paraffin, and paraffin, hydrophilic petrolatum, purified lanolin, absorption ointment, hydrous lanolin, hydrophilic ointment, starch, pullulan, gum arable, tragacanth gum, gelatin, dextran, cellulose derivatives such as methylcellulose, carboxymethyl cellulose, hydroxyethyl cellulose, and hydroxypropyl cellulose, synthetic polymers such as carboxyvinyl polymer, sodium polyacrylate, polyvinylalcohol, and polyvinylpyrrolidone, propylene glycol, macrogol such as Macrogol 200 to 600, and a combination of two or more of them.

Such a "preserving agent" includes, for example, ethyl parahydroxybenzoate, chlorobutanol, benzyl alcohol, sodium dehydroacetate, and sorbic acid.

Such an "anti-oxidant agent" includes, for example, sodium sulfite and ascorbic acid.

Such a "coloring agent" includes, for example, food colors (e.g., Food Red No. 2 or No. 3, Food Yellow No. 4, or No. 5) and β-carotene.

Such a "sweetening agent" includes, for example, saccharin sodium, dipotassium glycyrrhizinate, and aspartame.

A pharmaceutical composition herein may be administered orally or parenterally (e.g., topically, rectally, intravenously, intramuscularly, and subcutaneously) to humans as well as mammals other than humans such as mice, rats, hamsters, guinea pigs, rabbits, cats, dogs, pigs, cows, horses, sheep, and monkeys. Dosage amounts vary depending on subjects to be administered, diseases, conditions, dosage forms, and administration routes. For example, a daily dose for oral administration to an adult patient (60 kg of body weight) typically ranges from about 0.01 mg to about 1 g of the active ingredient. The dose can be administered at one time or in divided doses. In one embodiment, an SGLT1 inhibitor and other drugs may be formulated into separate pharmaceutical compositions, which may be used in combination and may be administered to a subject in any order and at any time interval in different administration routes. In another embodiment, a dosage amount of each drug in combination use may be reduced compared to administration of each drug alone, and the daily dose for oral administration to an adult patient (60 kg of body weight) ranges from about 0.01 mg to about 1000 mg.

In one embodiment, a kit such as kits for administration, treatment, and/or prevention, a package such as packaged goods, or a set and/or case of drugs which comprises an SGLT1 inhibitor, and optionally, an SGLT2 inhibitor, and a written matter concerning these drugs indicating that these drugs may or should be used for treatment and/or prevention may be provided. The kit, package, and set of drugs may comprise one or more containers filled with an SGLT1 inhibitor, and optionally an SGLT2 inhibitor, and/or other drugs or medicines (or ingredients). Examples of the kit, package, and set of drugs herein include commercial kits, commercial packages, and commercial medicine set for appropriate use in treatment and/or prevention of intended diseases. The written matter comprised in the kit, package, and set of drugs includes a cautionary note or package insert in the form designated by the government organization that regulates manufacturing, use, or sales of pharmaceutical or biological products which ensures an approval by the government organization on manufacturing, use, or sales of products associated with administration to humans. The kit, package, and set of drugs may include packaged products as well as structures configured for appropriate administration steps and configured so as to be able to achieve more preferable medical treatment and/or prevention including treatment and/or prevention of intended diseases.

### EXAMPLES

The compounds of Examples 1 to 40 (hereinafter referred to as Compounds 1 to 40) were obtained according to the preparation methods described in Patent Literatures 2 and 3. The physical properties and SGLT1 inhibitory activity data of each compound are as described in these literatures.

**[Table 3-1]**

| Example | Structure |
|---|---|
| 1 | 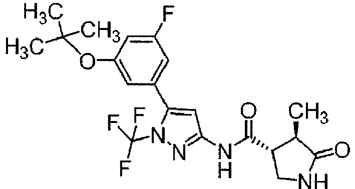 |
| 2 | 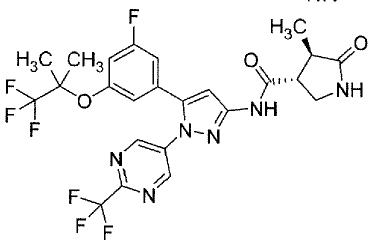 |
| 3 | 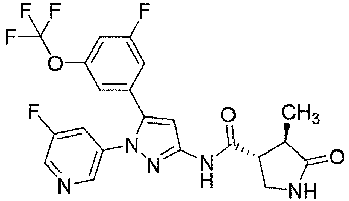 |

**[Table 3-2]**

| | |
|---|---|
| 4 | 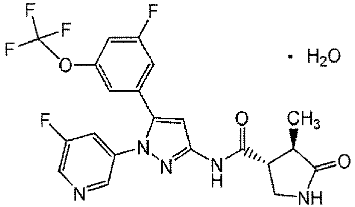 |
| 5 | 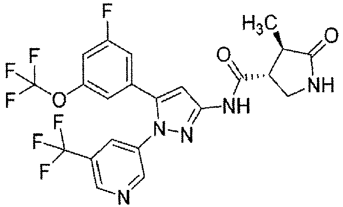 |
| 6 | 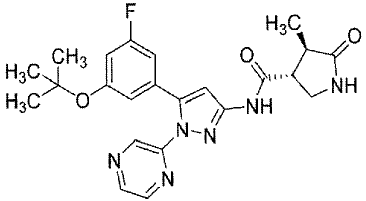 |
| 7 | 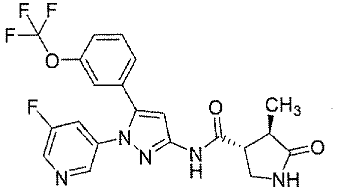 |
| 8 | 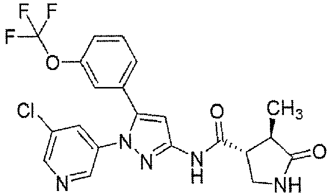 |
| 9 | 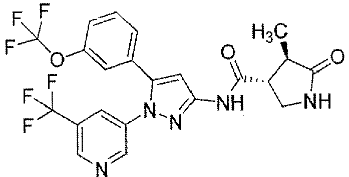 |

**[Table 3-3]**

| | |
|---|---|
| 10 | 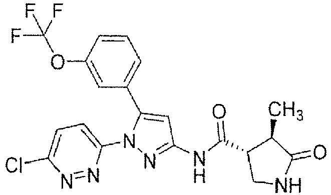 |
| 11 | 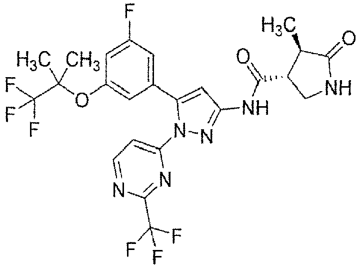 |
| 12 | 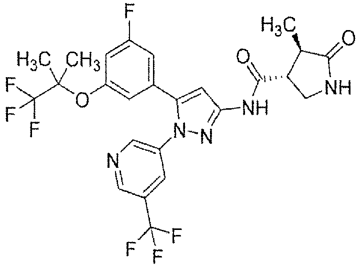 |
| 13 | 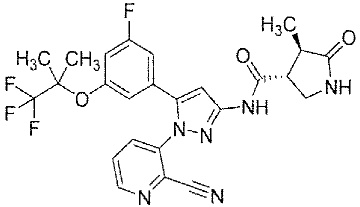 |
| 14 | 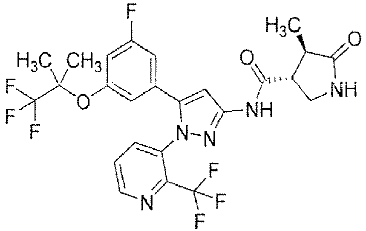 |

**[Table 3-4]**

| | |
|---|---|
| 15 | 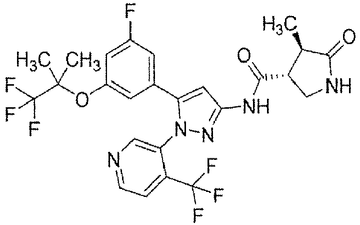 |
| 16 | 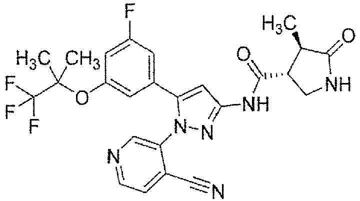 |
| 17 | 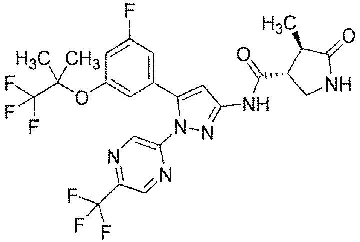 |
| 18 | 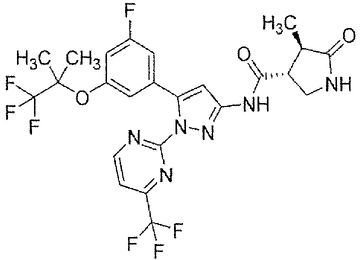 |
| 19 | 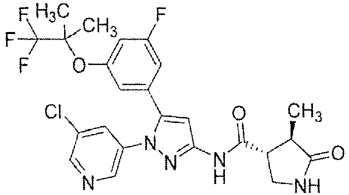 |

**[Table 3-5]**

| | |
|---|---|
| 20 | 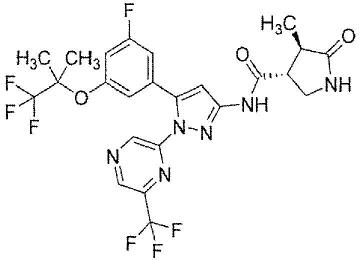 |
| 21 | 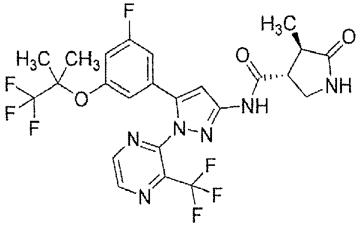 |
| 22 | 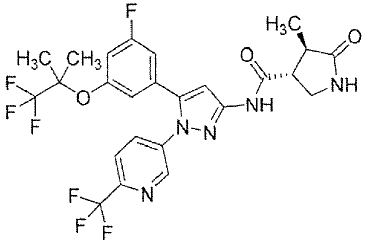 |
| 23 | 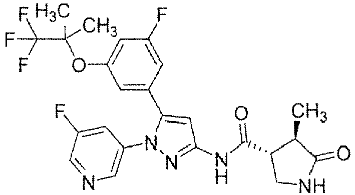 |
| 24 | 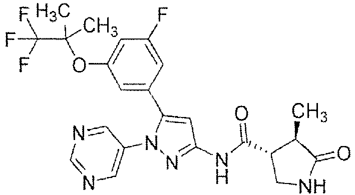 |

**[Table 3-6]**

| | |
|---|---|
| 25 | 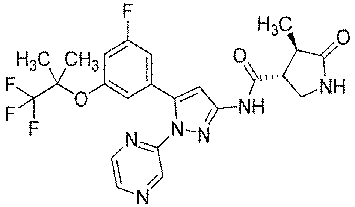 |
| 26 | 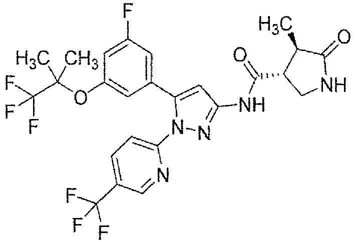 |
| 27 | 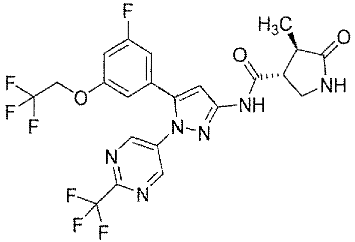 |
| 28 | 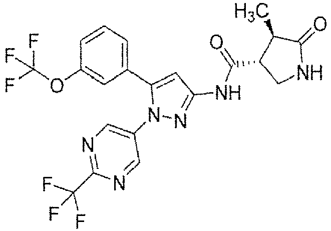 |
| 29 | 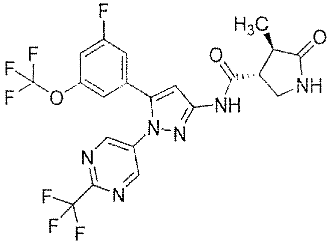 |

**[Table 3-7]**

| | |
|---|---|
| 30 | 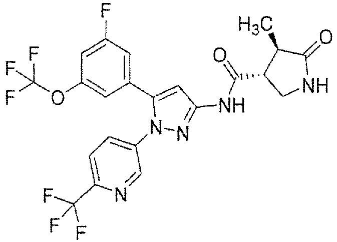 |
| 31 | 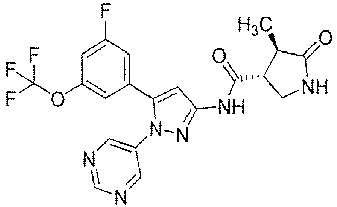 |
| 32 | 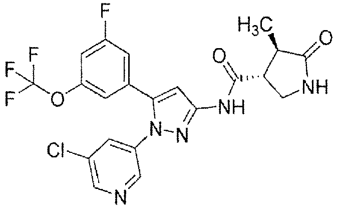 |
| 33 | 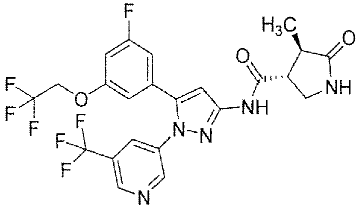 |
| 34 | 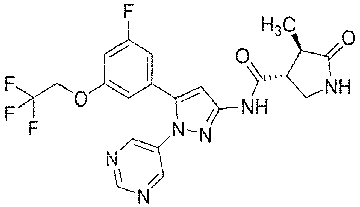 |

**[Table 3-8]**

| | |
|---|---|
| 35 | 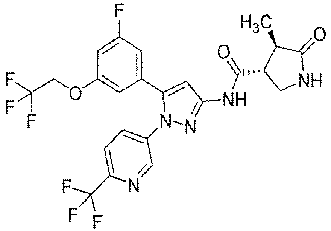 |
| 36 | 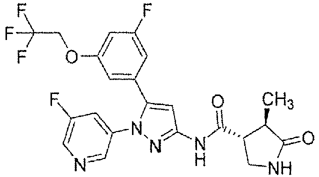 |
| 37 | 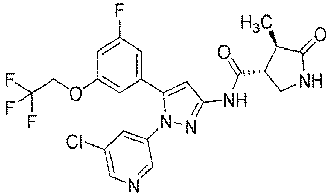 |
| 38 | 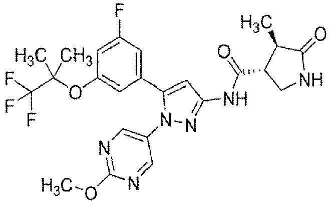 |
| 39 | 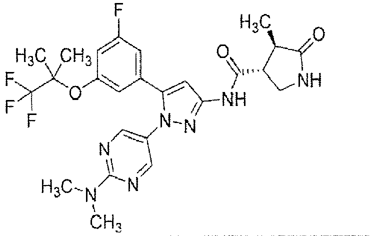 |
| 40 | 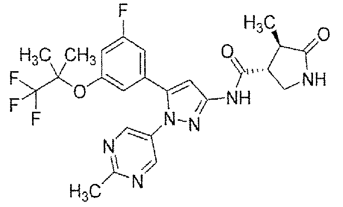 |

### [Test Example 1]

### Assessment of cardiac function in rats with post-myocardial infarction heart failure

Male SD rats (8-week old, Japan SLC, Inc.) were anesthetized with pentobarbital, then, under controlled mechanical ventilation, underwent thoracotomy and permanent ligation of the left anterior descending coronary artery to prepare rats with post-myocardial infarction heart failure. A sham-operated group was prepared by undergoing only thoracotomy in the same way to expose the heart. Two weeks later, vehicle (0.5% methylcellulose solution) or Compound 1 (3 mg/kg group or Ascending Dose group) was orally administered once daily to the group of post-myocardial infarction heart failure rat, and the vehicle was orally administered once daily to the sham-operated group. The dose for the Ascending Dose group was started with 3 mg/kg, increased to 6 mg/kg after two weeks and to 8 mg/kg after two more weeks, and maintained at 8 mg/kg thereafter. After eight weeks of administration, an ultrasonograph (Aplio 300, Toshiba Medical Systems Corporation) was used to measure the left ventricular ejection fraction, and Catheter-tipped micromanometer (Model SPR-320, Millar Inc.) was inserted into the left ventricle via the left carotid artery to measure the left ventricular end-diastolic pressure. For statistical analysis, Aspin-Welch t test was conducted between the sham-operated vehicle-administered group and the vehicle-administered group of post-myocardial infarction heart failure rats. Steel's multiple comparison test was conducted for the Compound 1 group to the vehicle group of post-myocardial infarction heart failure rats. The significance level was two-sided 5%. Form the results, it was confirmed that the vehicle-administered group of post-myocardial infarction heart failure rats developed HFrEF because the left ventricular ejection fraction was decreased and the left ventricular end-diastolic pressure was increased, compared with the sham-operated vehicle-administered group. In post-myocardial infarction heart failure rats, Compound 1 improved HFrEF because the left ventricular ejection fraction was increased, and the increase of the left ventricular end-diastolic pressure was suppressed. The results are shown in Figures 1 and 2.

It was confirmed by Test Example 1 that Compound 1 has an HFrEF improving effect. The HFrEF improving effect of Compounds 2 to 40 can be confirmed by a method similar to Test Example 1. The HFrEF improving effect of Compounds 1 to 40 (for example, Compound 1) in combination with an SGLT2 inhibitor (for example, dapagliflozin) can be confirmed by a method similar to Test Example 1.

### [Test Example 2]

### Assessment of cardiac function in rats with hypertensive heart failure

Male DIS/Eis (Dahl-Iwai S) rats (Dahl rats) (7-week old, Japan SLC, Inc.) were fed a high-salt diet (MF 8% sodium chloride-adjusted feed, Oriental Yeast Co., ltd.) to prepare hypertensive heart failure rats. Normal controls were fed a normal diet (CRF-1 solid, Oriental Yeast Co., ltd.). Three weeks later, after confirmation of the development of pathological condition (hypertension or cardiomegaly), vehicle (0.5% methylcellulose solution) or Compound 1 (3 mg/kg group or Ascending Dose group) was orally administered to the hypertensive heart failure rat group once daily, and the vehicle was orally administered once daily to the normal control group. The dose for the Ascending Dose group was started with 3 mg/kg, increased to 6 mg/kg after a week, gradually increased to 8 mg/kg in the next week, and maintained at 8 mg/kg thereafter. After five weeks of administration, an ultrasonograph (Aplio 300, Toshiba Medical Systems Corporation) was used to measure the left ventricular ejection fraction, and a non-invasive automated blood pressure measuring device (BP-98A, Softlon Co.,Ltd.) was used to measure the systolic blood pressure. After six weeks of administration, ADVantage P-V catheter (Model FTH-1918B-E318, Transonic Scisense Inc.) was inserted into the left ventricle from the apex to measure the left ventricular end-diastolic pressure. In addition, ADVantage P-V catheter and Ultrasonic flow probe (Model 2.5PSB1459, Transonic Scisense Inc.) were used to calculate EDPVRβ from the pressure-volume relationship due to the transient inferior vena cava occlusion. For statistical analysis, Student test or Aspin-Welch t test was conducted between the vehicle-administered normal control group and the vehicle-administered group of hypertensive heart failure rats. Dunnett's multiple test was conducted for the Compound 1 group to the vehicle group of hypertensive heart failure rats. The significance level was two-sided 5%. Form the results, it was confirmed that the hypertensive heart failure rats developed HFpEF because the EDPVRβ and left ventricular end-diastolic pressure were increased whereas the left ventricular ejection fraction was not decreased, compared with the normal control group. Furthermore, in the hypertensive heart failure rats, Compound 1 reduced the EDPVRβ and left ventricular end-diastolic pressure, thereby improving HFpEF. The hypertensive heart failure rats also showed an increase of systolic blood pressure compared with the normal control group, thereby developing hypertension, but Compound 1 had no effect on the systolic blood pressure. Compound 1 also had no effect on the urine volume in the hypertensive heart failure rats. The results are shown in Figures 3, 4, 5, 6 and 7.

It was confirmed by Test Example 2 that Compound 1 has an HFpEF improving effect. The HFpEF improving effect of Compounds 2 to 40 can be confirmed by a method similar to Test Example 2. The HFpEF improving effect of Compounds 1 to 40 (for example, Compound 1) in combination with an SGLT2 inhibitor (for example, dapagliflozin) can be confirmed by a method similar to Test Example 2.

### [Formulation Examples]

Formulation Examples of a compound of Formula [I] include, for example, the following formulations, but are not intended to be limited thereto.

### Formulation Example 1 (Preparation of a capsule)

| | |
|---|---|
| (1) Compound 1 | 30 mg |
| (2) Microcrystalline cellulose | 10 mg |
| (3) Lactose | 19 mg |
| (4) Magnesium stearate | 1 mg |

Ingredients (1), (2), (3), and (4) are mixed to be filled in a gelatin capsule.

### Formulation Example 2 (Preparation of a tablet)

| | |
|---|---|
| (1) Compound 1 | 10 g |
| (2) Lactose | 50 g |
| (3) Cornstarch | 15 g |
| (4) Carmellose calcium | 44 g |
| (5) Magnesium stearate | 1 g |

The total amount of Ingredients (1), (2), and (3) and 30 g of Ingredient (4) are combined with water, dried in vacuo, and then granulated. The resulted granules are mixed with 14 g of Ingredient (4) and 1 g of Ingredient (5), and tableted with a tableting machine. In this manner, 1000 tablets comprising 10 mg of Compound 1 for each tablet are obtained.

### INDUSTRIAL APPLICABILITY

A compound inhibiting SGLT1, or a pharmaceutically acceptable salt thereof, is expected to be useful for treatment or prevention of chronic heart failure.

## Claims

1. A pharmaceutical composition for treatment or prevention of chronic heart failure, comprising a compound inhibiting SGLT1 or a pharmaceutically acceptable salt thereof.

2. A pharmaceutical composition for treatment or prevention of chronic heart failure, comprising a compound of Formula [I]: 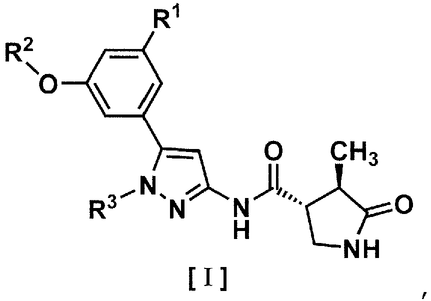 or a pharmaceutically acceptable salt thereof,
wherein R¹ is hydrogen or halogen;
R² is C₁₋₆ alkyl or halo-C₁₋₆ alkyl;
R³ is
(1) C₁₋₆ alkyl,
(2) halo-C₁₋₆ alkyl,
(3) pyridyl substituted with R^{3A}, or
(4) pyrazinyl, pyrimidinyl, or pyridazinyl, which may be optionally substituted with R^{3B};
R^{3A} is cyano, halogen, or halo-C₁₋₃ alkyl;
R^{3B} is halogen, hydroxy, C₁₋₃ alkyl, halo-C₁₋₃ alkyl, C₁₋₃ alkoxy, or -N(R⁴)(R⁵); and
R⁴ and R⁵ are each independently hydrogen or C₁₋₃ alkyl.

3. The pharmaceutical composition according to claim 1 or 2, wherein the compound inhibiting SGLT1 or the compound of Formula [I] or a pharmaceutically acceptable salt thereof is any one of compounds of Formulae [II] to [V]: 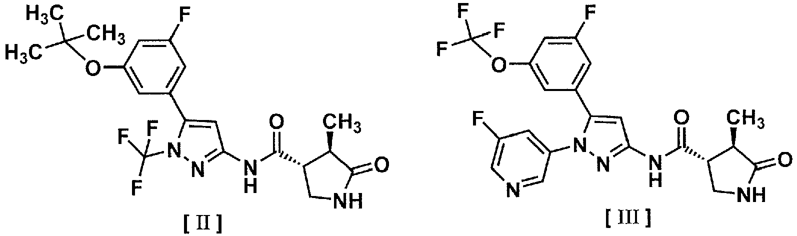 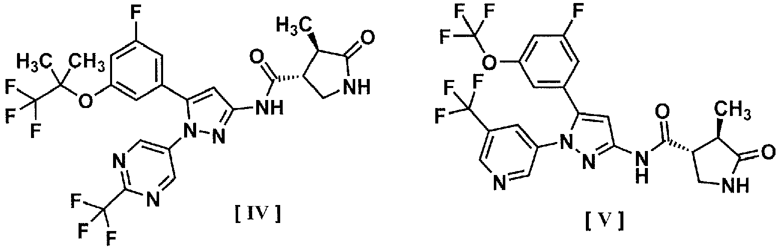 or a pharmaceutically acceptable salt thereof.

4. The pharmaceutical composition according to any one of claims 1 to 3, wherein the compound inhibiting SGLT1 or the compound of Formula [I] or a pharmaceutically acceptable salt thereof is a compound of Formula [II]: 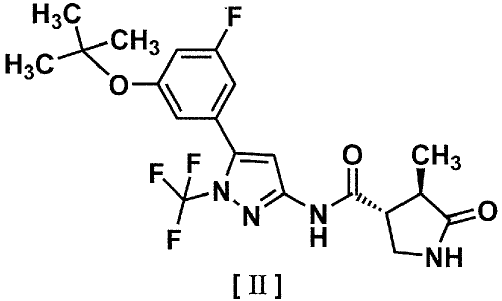 or a pharmaceutically acceptable salt thereof.

5. The pharmaceutical composition according to any one of claims 1 to 4, wherein the chronic heart failure is HFrEF.

6. The pharmaceutical composition according to any one of claims 1 to 4, wherein the chronic heart failure is HFpEF.

7. A method of treating or preventing chronic heart failure, comprising administering a therapeutically effective amount of a compound inhibiting SGLT1 or a compound of Formula [I] according to claim 2 or a pharmaceutically acceptable salt thereof, to a subject.

8. A compound inhibiting SGLT1 or a compound of Formula [I] according to claim 2 or a pharmaceutically acceptable salt thereof, for use in the treatment or prevention of chronic heart failure.

9. Use of a compound inhibiting SGLT1 or a compound of Formula [I] according to claim 2 or a pharmaceutically acceptable salt thereof, in the manufacture of a medicament for treatment or prevention of chronic heart failure.
